# EUROPEAN PATENT SPECIFICATION

(11) **EP 0 427 404 B1**
(45) Date of publication and mention of the grant of the patent: **25.05.1994**
(21) Application number: 90311211.8
(22) Date of filing: 12.10.1990
(51) Int. Cl.: C07C 323/22, G03G 5/06, C07D 215/36

(54) **Anthanthrone derivatives**
Anthantron-Derivate
Dérivés d'anthantrone

(30) Priority: 09.11.1989 GB 8925362
(43) Date of publication of application: 15.05.1991
(73) Proprietor: ZENECA LIMITED, London W1Y 6LN (GB)
(72) Inventor: Mistry, Pralad, Lancashire OL6 8XU (GB); Patel, Prakash, Huddersfield HD3 3AX (GB)
(74) Representative: Pugsley, Roger Graham

(56) References cited:
- CH-A- 452 755
- FR-A- 1 308 796

## Description

This invention relates to a novel anthanthrone compound which may be used as a charge generating compound (CGC) in the photosensitive elements of an electrophotographic device such as a copier or printer.

FR 1,308,796 discloses diphenylthioanthanthrone obtained from dibromoanthanthrone and thiophenol and its use for dyeing cotton in violet shades.

According to the present invention there is provided a compound of general Formula (1): 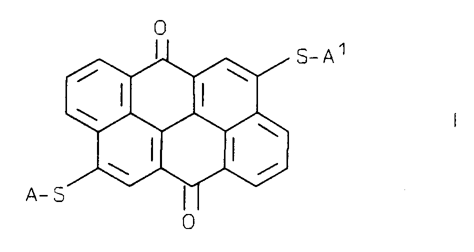
wherein
- A and A¹: are each independently selected from phenyl, substituted phenyl, quinolino, naphthyl and substituted naphthyl wherein the substituents are independently selected from halo, alkyl, alkoxy, alkylthio, phenyl, phenoxy, phenylthio, CF₃, CH₂OH, CO₂R¹, NHCOR¹, COR¹, NO₂ and NR¹R²;
- R¹ and R²: are each independently H or C₁₋₆-alkyl; with the proviso that A and A¹ are not both phenyl.

It is preferred that A and A¹ are each independently phenyl or naphthyl having up to three substituents, especially up to two substituents, more especially none or a single substituent.

When either or both of A and A¹ are substituted phenyl or substituted naphthyl it is preferred that the substituents are independently selected from C₁₋₆alkyl, C₁₋₆-alkoxy, phenyl, fluoro, chloro, bromo and NR¹R² wherein R¹ and R² are preferably each independently C₁₋₄-alkyl.

It is further preferred that the optional substituents on A and A¹ are independently selected from C₁₋₄-alkyl, C₁₋₄-alkoxy, chloro, bromo, fluoro, phenyl, NHCOR¹ and CO₂R¹, wherein R¹ is C₁₋₄-alkyl especially C₁₋₄-alkyl, C₁₋₄-alkoxy, Cl and Br. It is most preferred that the optional substituents on A and A¹ are independently selected from methyl, methoxy, chloro and bromo.

It is further preferred that A and A¹ are each independently selected from naphthyl and 4-methylphenyl and more especially that A is 4-methylphenyl and A¹ is naphth-2-yl.

A CGC used in known photosensitive element of electrophotographic devices is dibromoanthanthrone (DBA) as a CGC which has an absorption maxima in dimethyl formamide at 536nm.

It has been found that compounds of the present invention have absorption maxima at longer wavelengths which more closely match the spectral output of lamps commonly used in electrophotographic devices. Thus the present compounds generally have a better photographic response than DBA in such devices.

Although the present compounds are beneficial on their own as CGCs, in admixture with DBA they provide compositions which can be used to extend the spectral response of a photosensitive element. The compositions may comprise a physical mixture of the compounds or a mixed crystal formed by co-precipitation of the two components from solution.

Extension of the spectral response is important in monochrome copying to enhance the intensity of black copies of originals printed in magenta through to green colours and in colour copying to give a more even response across the visible spectrum and thereby more faithful reproduction of the whole spectrum of colours.

The compounds of the present invention may generally be prepared by reacting an aromatic thiol with dibromoanthanthrone in the presence of a base. The reaction is preferably carried out in an organic aprotic solvent, by reacting the thiol with the base (from the thiol anion) followed by reaction with dibromoanthanthrone.

Symmetrical compounds, i.e. those in which A and A¹ are the same, may be prepared by reacting an excess of the appropriate thiol anion with DBA, preferably at least 2 moles/mole.

Unsymmetrical compounds can be prepared by reacting the DBA with at least 1 mole/mole of a first thiol, separating out the mono thiol adduct and then reacting this with at least 1 mole/mole of a second thiol. Alternatively, the DBA may be reacted with a mixture of the two thiols to give a mixture comprising two symmetrical adducts and one unsymmetrical adduct. Such mixtures may be used as CGC's in the form of a mixture or separated using known chromatographic techniques into the component compounds.

The compounds of Formula I and mixtures thereof can be used alone or in admixture with DBA in all proportions in place of DBA or any other CGC in the photosensitive element of an electrophotographic device.

The following examples are given as illustrations of the present invention.

### Example 1

### Compound of Formula (1) wherein A=A¹=4-methylphenyl

A mixture of p-thiocresol (10g; 0.08mol) and potassium hydroxide (5g; 0.08mol) in DMF (100ml) was stirred at 60°C in an ultrasonic bath for 1 hour. Dibromoanthanthrone (9.3g; 0.02mol) was then added and the mixture stirred for a further 1 hour at 60°C. The reaction mixture was cooled to 50°C and added to methanol (1 litre). The resultant slurry was filtered and the solid washed with water and dried to give the title compound (9g, 82%). λmax (DMF) 566nm.

The compounds of Formula (1) in which A and A¹ are the same and have the meanings defined in the first column of Table 1 may be prepared by the method of Example 1, but in each case, replacing the 10g p-thiocresol by 10g of the thiol identified in the second column of Table 1.

**Table 1**

| Example | A = A¹ | Thiol |
|---|---|---|
| 2 | 3-methylphenyl | m-thiocresol |
| 3 | 2-methylphenyl | o-thiocresol |
| 4 | 2-chlorophenyl | 2-chlorophenylthiol |
| 5 | 3-chlorophenyl | 3-chlorophenylthiol |
| 6 | 4-chlorophenyl | 4-chlorophenylthiol |
| 7 | 2-methoxyphenyl | 2-methoxyphenylthiol |
| 8 | 3-methoxyphenyl | 3-methoxyphenylthiol |
| 9 | 4-methoxyphenyl | 4-methoxyphenylthiol |
| 10 | 2,4-dichlorophenyl | 2,4-dichlorophenylthiol |
| 11 | 2,5-dichlorophenyl | 2,5-dichlorophenylthiol |
| 12 | 2,6-dichlorophenyl | 2,6-dichlorophenylthiol |
| 13 | 2,4,6-trichlorophenyl | 2,4,6-trichlorophenylthiol |
| 14 | 3,4-dimethylphenyl | 3,4-dimethylphenylthiol |

### Example 15

### Compound of Formula (1) wherein A=4-methylphenyl and A¹=naphth-2-yl

### Stage I

A mixture of 2-thionaphthol (10g; 0.06mol) and potassium hydroxide (3.36g; 0.06mol) in DMF (100ml) was stirred at 60°C for 45 minutes. Dibromoanthanthrone (14.5g; 0.03mol) was then added and the mixture stirred at 60°C for 2 hours. The reaction mixture was cooled to 50°C and added to methanol (1 litre). The resultant slurry was filtered and the solid washed with water, dried to give a violet pigment (17.9g; 92%), bromo(thionaphth-2-yl)-anthanthrone. λmax (DMF) 540nm.

### Stage II

A mixture of 4-thiocresol (4g; 0.03mol) and potassium hydroxide (1.8g; 0.03mol) in DMF (150ml) was stirred at 60°C for 1 hour. Bromo-(thionaphth-2-yl)anthanthrone (10g; 0.016mol) from Stage I was then added and the mixture stirred at 60°C for 4 hours. The reaction mixture was cooled to 50°C and added to methanol (1 litre). The resultant slurry was filtered and the solid washed with water, dried to give the title compound (8.45g, 79%). λmax (DMF) 556nm.

The compounds of Formula (1) in which A¹ = naphth-2-yl and A has the meaning defined in the first column of Table 2 may be prepared by the method of Examples 15, in each case replacing the 4g of 4-thiocresol by 4g of the thiol identified in the second column of Table 2.

**Table 2**

| Example | A (A¹ = naphth-2-yl) | Thiol |
|---|---|---|
| 16 | 3-methylphenyl | m-thiocresol |
| 17 | 2-methylphenyl | o-thiocresol |
| 18 | phenyl | thiophenol |
| 19 | 2-chlorophenyl | 2-chlorophenylthiol |
| 20 | 3-chlorophenyl | 3-chlorophenylthiol |
| 21 | 4-chlorophenyl | 4-chlorophenylthiol |
| 22 | 2-methoxyphenyl | 2-methoxyphenylthiol |
| 23 | 3-methoxyphenyl | 3-methoxyphenylthiol |
| 24 | 4-methoxyphenyl | 4-methoxyphenylthiol |
| 25 | 2,4-dichlorophenyl | 2,4-dichlorophenylthiol |
| 26 | 2,5-dichlorophenyl | 2,5-dichlorophenylthiol |
| 27 | 2,6-dichlorophenyl | 2,6-dichlorophenylthiol |
| 28 | 2,4,6-trichlorophenyl | 2,4,6-trichlorophenylthiol |
| 29 | 3,4-dimethylphenyl | 3,4-dimethylphenylthiol |

The compounds of Formula (1) in which A¹ is phenyl and A has the meaning defined in the first column of Table 3 may be prepared by the method of Example 16, in each case replacing 2-thionaphthol by a molecular equivalent of phenylthiol, and replacing the 4g of 4-thiocresol by 4g of the thiol identified in the second column of Table 3.

**Table 3**

| Example | A (A¹ = phenyl) | Thiol |
|---|---|---|
| 30 | 3-methylphenyl | m-thiocresol |
| 31 | 2-methylphenyl | o-thiocresol |
| 32 | 2-chlorophenyl | 2-chlorophenylthiol |
| 33 | 3-chlorophenyl | 3-chlorophenylthiol |
| 34 | 4-chlorophenyl | 4-chlorophenylthiol |
| 35 | 2-methoxyphenyl | 2-methoxyphenylthiol |
| 36 | 3-methoxyphenyl | 3-methoxyphenylthiol |
| 37 | 4-methoxyphenyl | 4-methoxyphenylthiol |
| 38 | 2,4-dichlorophenyl | 2,4-dichlorophenylthiol |
| 39 | 2,5-dichlorophenyl | 2,5-dichlorophenylthiol |
| 40 | 2,6-dichlorophenyl | 2,6-dichlorophenylthiol |
| 41 | 2,4,6-trichlorophenyl | 2,4,6-trichlorophenylthiol |
| 42 | 3,4-dimethylphenyl | 3,4-dimethylphenylthiol |

### Example 43

### Compound of Formula (1) wherein A = 3-methoxyphenyl and A¹ = 4-chlorophenyl

The method of Example 15 can be followed except that in place of 2-thionaphthol there is used an equivalent amount of 3-methoxyphenylthiol and in place of 4-thiocresol there is used an equivalent amount of 4-chlorophenylthiol.

## Claims

1. A compound of the formula: 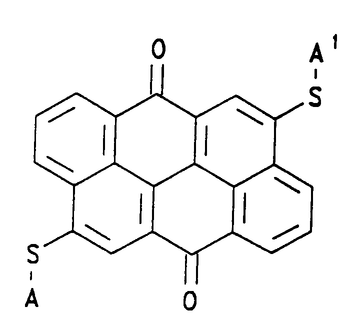 wherein:
A and A¹ are each independently selected from phenyl, substituted phenyl, quinolino, naphthyl and substituted naphthyl wherein the substituents are independently selected from halo, alkyl, alkoxy, alkylthio, phenyl, phenoxy, phenylthio, CF₃, CH₂OH, CO₂R¹, NHCOR¹, COR¹, NO₂ and NR¹R²;
R¹ and R² are each independently H or C₁₋₆-alkyl; with the proviso that A and A¹ are not both phenyl.

2. A compound according to claim 1 in which A and A¹ are each independently selected from phenyl and naphthyl each independently having up to three substituents.

3. A compound according to Claim 1 or Claim 2 in which R¹ and R² are each independently C₁₋₄-alkyl.

4. A compound according to Claim 1 or Claim 2 in which said substituents are selected from C₁₋₄-alkyl, C₁₋₄-alkoxy, chloro, bromo, fluoro, phenyl, NHCOR¹ and CO₂R¹.

5. A compound according to claim 1 or claim 2 in which the substituents are selected from C₁₋₄-alkyl or C₁₋₄-alkoxy.

6. A compound according to claim 1 or claim 2 in which the substituents are selected from methyl, methoxy, chloro and bromo.

7. A compound according to claim 1 in which A and A¹ are each independently selected from naphthyl and 4-methylphenyl.

8. A compound according to claim 1 in which A¹ is naphth-2-yl and A is 4-methylphenyl.

9. A composition comprising dibromanthranthrone and a compound of formula I: 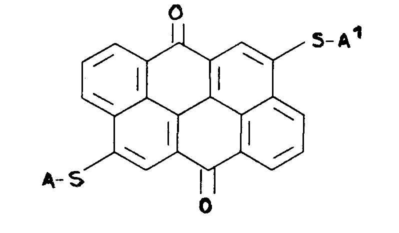 wherein
A and A¹ are each independently selected from phenyl, substituted phenyl, quinolino, naphthyl and substituted naphthyl wherein the substituents are independently selected from halo, alkyl, alkoxy, alkylthio, phenyl, phenoxy, phenylthio, CF₃, CH₂OH, CO₂R¹, NHCOR¹, COR¹, NO₂ and NR¹R²;
R¹ and R² are each independently H or C₁₋₆-alkyl.

10. A composition according to claim 9 in the form of a mixed crystal.

11. The use of a compound of formula (I) or a composition according to any preceding claim as a charge generating compound in a photosensitive element of an electrophotographic device; 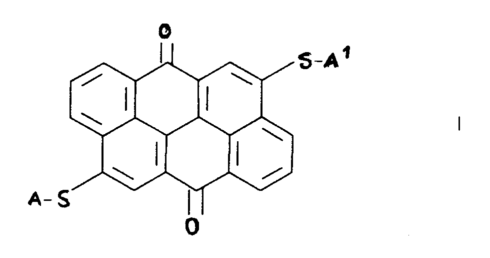 wherein
A and A¹ are each independently selected from phenyl, substituted phenyl, quinolino, naphthyl and substituted naphthyl wherein the substituents are independently selected from halo, alkyl, alkoxy, alkylthio, phenyl, phenoxy, phenylthio, CF₃, CH₂OH, CO₂R¹, NHCOR¹, COR¹, NO₂ and NR¹R²;
R¹ and R² are each independently H or C₁₋₆-alkyl.

## Patentansprüche

1. Verbindungen der Formel 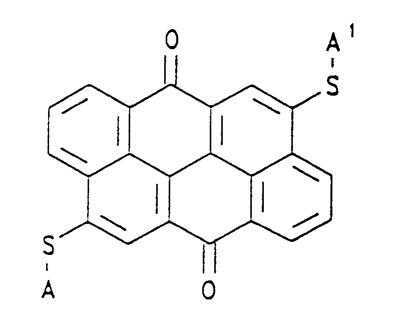 worin
A und A¹ jeweils unabhängig ausgewählt sind aus Phenyl, substituiertem Phenyl, Chinolino, Naphthyl und substituiertem Naphthyl, wobei die Substituenten unabhängig ausgewählt sind aus Halogeno, Alkyl, Alkoxy, Alkylthio, Phenyl, Phenoxy, Phenylthio, CF₃, CH₂OH, CO₂R¹, NHCOR¹, COR¹, NO₂ und NR¹R²; und
R¹ und R² jeweils unabhängig ausgewählt sind aus H und C₁₋₆-Alkyl;
mit der Maßgabe, daß A und A¹ nicht beide für Phenyl stehen.

2. Verbindungen nach Anspruch 1, worin A und A¹ jeweils unabhängig ausgewählt sind aus Phenyl und Naphthyl, die jeweils unabhängig bis zu drei Substituenten aufweisen.

3. Verbindungen nach Anspruch 1 oder 2, worin R¹ und R² jeweils unabhängig für C₁₋₄-Alkyl stehen.

4. Verbindungen nach Anspruch 1 oder 2, worin die Substituenten ausgewählt sind aus C₁₋₄-Alkyl, C₁₋₄-Alkoxy, Chloro, Bromo, Fluoro, Phenyl, NHCOR¹ und CO₂R¹.

5. Verbindungen nach Anspruch 1 oder 2, worin die Substituenten ausgewählt sind aus C₁₋₄-Alkyl und C₁₋₄-Alkoxy.

6. Verbindungen nach Anspruch 1 oder 2, worin die Substituenten ausgewählt sind aus Methyl, Methoxy, Chloro und Bromo.

7. Verbindungen nach Anspruch 1, worin A und A¹ jeweils unabhängig ausgewählt sind aus Naphthyl und 4-Methylphenyl.

8. Verbindung nach Anspruch 1, worin A¹ für Naphth-2-yl und A für 4-Methylphenyl steht.

9. Zusammensetzung, welche ein Dibromoanthanthron und eine Verbindung der Formel I 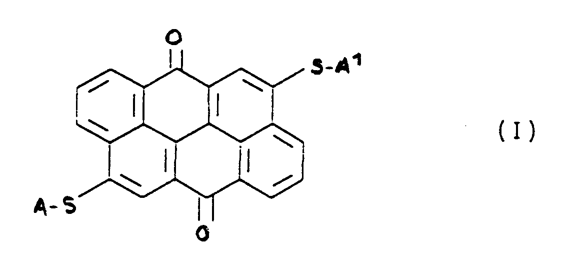 enthält, worin
A und A¹ jeweils unabhängig ausgewählt sind aus Phenyl, substituiertem Phenyl, Chinolino, Naphthyl und substituiertem Naphthyl, wobei die Substituenten unabhängig ausgewählt sind aus Halogeno, Alkyl, Alkoxy, Alkylthio, Phenyl, Phenoxy, Phenylthio, CF₃, CH₂OH, CO₂R¹, NHCOR¹, COR¹, NO₂ und NR¹R²; und
R¹ und R² jeweils unabhängig für H oder C₁₋₆-Alkyl stehen.

10. Zusammensetzung nach Anspruch 9 in Form eines Mischkristalls.

11. Die Verwendung einer Verbindung der Formel I oder einer Zusammensetzung nach einem der vorhergehenden Ansprüche als Ladungserzeugungsverbindung in einem fotosensitiven Element einer elektrofotografischen Vorrichtung 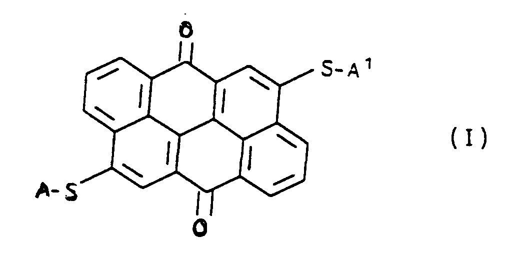 worin
A und A¹ jeweils unabhängig ausgewählt sind aus Phenyl, substituiertem Phenyl, Chinolino, Naphthyl und substituiertem Naphthyl, wobei die Substituenten unabhängig ausgewählt sind aus Halogeno, Alkyl, Alkoxy, Alkylthio, Phenyl, Phenoxy, Phenylthio, CF₃, CH₂OH, CO₂R¹, NHCOR¹, COR¹, NO₂ und NR¹R²; und
R¹ und R² jeweils unabhängig für H oder C₁₋₆-Alkyl stehen.

## Revendications

1. Composé de formule : 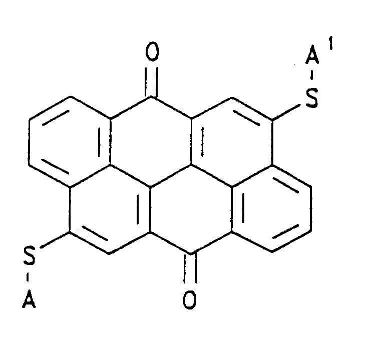 dans laquelle :
A et A¹ sont choisis chacun indépendamment entre des groupes phényle, phényle substitué, quinolino, naphtyle et naphtyle substitué dans lesquels les substituants sont choisis indépendamment entre des substituants halogéno, alkyle, alkoxy, alkylthio, phényle, phénoxy, phénylthio, CF₃, CH₂OH, CO₂R¹, NHCOR¹, COR¹, NO² et NR¹R² ;
R¹ et R² représentent chacun indépendamment H ou un groupe alkyle en C₁ à C₆ ; sous réserve que A et A' ne représentent pas l'un et l'autre un groupe phényle.

2. Composé suivant la revendication 1, dans lequel A et A¹ sont choisis chacun indépendamment entre des groupes phényle et naphtyle possédant chacun indépendamment jusqu'à trois substituants.

3. Composé suivant la revendication 1 ou la revendication 2, dans lequel R¹ et R² représentent chacun indépendamment un groupe alkyle en C₁ à C₄.

4. Composé suivant la revendication 1 ou la revendication 2, dans lequel les substituants sont choisis entre des substituants alkyle en C₁ à C₄, alkoxy en C₁ à C₄, chloro, bromo, fluoro, phényle, NHCOR¹ et CO₂R¹.

5. Composé suivant la revendication 1 ou la revendication 2, dans lequel les substituants sont choisis entre des groupes alkyle en C₁ à C₄ ou alkoxy en C₁ à C₄.

6. Composé suivant la revendication 1 ou la revendication 2, dans lequel les substituants sont choisis entre des substituants méthyle, méthoxy, chloro et bromo.

7. Composé suivant la revendication 1, dans lequel A et A¹ sont choisis chacun indépendamment entre des groupes naphtyle et 4-méthylphényle.

8. Composé suivant la revendication 1, dans lequel A¹ représente un groupe naphtoyle et A représente un groupe 4-méthylphényle.

9. Composé comprenant de la dibromanthanthrone et un composé de formule I : 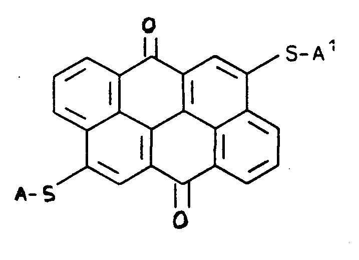 dans laquelle
A et A¹ sont choisis chacun indépendamment entre des groupes phényle, phényle substitué, quinolino, naphtyle et naphtyle substitué, dans lesquels les substituants sont choisis indépendamment entre des substituants halogéno, alkyle, alkoxy, alkylthio, phényle, phénoxy, phénylthio, CF₃, CH₂OH, CO₂R¹, NHCOR¹, COR¹, NO₂ et NR¹R² ;
R¹ et R² représentent chacun indépendamment H ou un groupe alkyle en C₁ à C₆.

10. Composition suivant la revendication 9, sous forme d'un cristal mixte.

11. Utilisation d'un composé de formule (I) ou d'une composition suivant l'une quelconque des revendications précédentes comme composé de production de charge dans un élément photosensible d'un dispositif électrophotographique 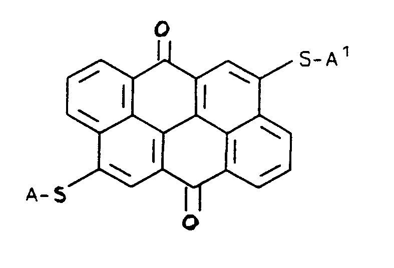 formule dans laquelle
A et A¹ sont choisis chacun indépendamment entre des groupes phényle, phényle substitué, quinolino, naphtyle et naphtyle substitué, dans lesquels les substituants sont choisis chacun indépendamment entre des substituants halogéno, alkyle, alkoxy, alkylthio, phényle, phénoxy, phénylthio, CF₃, CH₂OH, CO₂R¹, NHCOR¹, COR¹, NO₂ et NR¹R² ;
R¹ et R² représentent chacun indépendamment H ou un groupe alkyle en C₁ à C₆.
